# EUROPEAN PATENT APPLICATION

(11) **EP 0 639 387 A1**
(43) Date of publication of application: **22.02.1995**
(21) Application number: 93201857.5
(22) Date of filing: 25.06.1993
(51) Int. Cl.: A61M 36/12, A01K 11/00

(54) **Clamping means for an injector**

(71) Applicant: TEXAS INSTRUMENTS INCORPORATED, Dallas Texas 75265 (US); Texas Instruments Holland B.V., NL-7600 AA Almelo (NL)
(72) Inventor: Benning, Franciscus, Hendrikus, Cornelis, NL-7609 JS ALMELO (NL); van der Aa, Bartholomeus, Wilhelmus Johannes, NL-2611 LB Delft (NL)
(74) Representative: de Bruijn, Leendert C.

(57) **Abstract**

Injector for introducing solid objects (10), such as transponders, in living beings. To overcome the problem that the transponders must be kept in correct position near the extremity of the hollow needle of the injector clamping means are provided in the hollow needle or tube to engage the article. These clamping means (4) are realized such that the push rod (3) is not restricted in its movement whilst it is relatively easy to move the transponder out of engagement from the clamping means by this push rod.

## Description

The subject invention relates to an injector for introducing solid objects, such as transponders, in living beings, comprising a hollow tube for receiving the object and a push rod displaceable in said hollow tube to transfer the object to be injected.

Such an injector is generally known in the art. E.g. reference is made to the Netherlands patent application 9100160. In this patent specification an injector is described in which a push rod removes transponders from a cartridge in a hollow tube. This tube has to be inserted under the skin of a living being after which either by displacing of the push rod or by retracting of the needle or by a combined movement the transponder is placed in position.

However, it has been noticed that problems arise during transfer of the injector after the push rod has removed one of the transponders from the cartridge. The transponder can move freely in front of the push rod. Also in other embodiments wherein no cartridge is used, but instead of that the transponder is simply placed from the front end of the hollow needle in abutment with the transponder there is a substantial danger that the transponder gets astray during movement of the injector.

It has been proposed to use an ointment to keep the transponder engaged within the hollow needle or tube. Although this will prevent getting astray of the transponder it will also result in that ointment will remain in the tube and can absorb dirt from the surroundings and germs of a disease and so on from the animal which can be subsequently transferred to the next animal which has to be provided with a transponder. To prevent this it is necessary to clean the needle tip after each injection or to odd strongly disinfecting components to the ointments which will, however, decrease its effect during injection in the related animal.

Instead of using an ointment it is desirable to use a liquid. The chances that residues remain are less and it will not attract the surrounding dirt. Furthermore some medications are only available in liquid condition and not as ointment.

The invention aims to provide a solution for the problem to keep the transponder in its position within the hollow injector tube and being free whether or not to add any ointment, liquid or nothing at all.

According to the invention it is realized with an injector of the type described above in that clamping means are provided in the hollow tube to engage the object. According to the invention the object is kept mechanically in its position independent of any metered addition made to the transponder.

Such clamping means can comprise all clamping means known in the art. Preferably resilient means are provided arranged the near circumference of the hollow injector tube. These resilient means can be all kind of spring, resilient material and so on.

However, preferably a sheet of resilient material is used and more preferably in the shape of an elongated lip. The longitudinal axis of this lip should preferably be parallel to the longitudinal axis of the injector tube. Of course it is necessary to provide mounting means to connect the lip to the tube. These mounting means can also be a part of the sheet material from which the lip is realised and being substantially U-shaped in the mounted position in the tube. To have a guarantee that the related object is kept engaged within the tube as long as possible, the mounting means are preferably arranged upstream from the lip in the injector tube. Preferably the inner diameter within the mounting means is larger than the outer diameter of the pushing rod. The hollow injector tube can be provided with a sharp free extremity which acts as needle to intrude within living being.

The invention will be further elucidated according to a preferred embodiment of the invention which is further elucidated below and shown in the appended drawing, and wherein:
Fig. 1 shows diagrammatically an injector according to the prior art provided with the clamping means of the invention;
Fig. 2 partially in cross section a detail of the tip of the injector according to Fig. 1;
Fig. 3 a cross section along line III-III in Fig. 2;
Fig. 4 a cross section according to line IV-IV in Fig. 2 and
Fig. 5 a sheet of resilient material to be introduced in the hollow needle to act as clamping means.

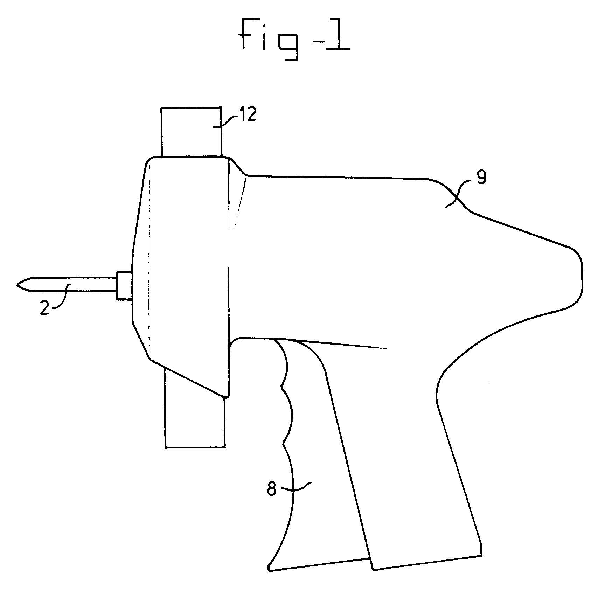

In Fig. 1 an injector is generally denoted with 1. The internals of this injector are not further shown and not of any importance of the subject invention, but for details for such embodiments and its functioning reference is made to the Netherlands patent application 9100160.

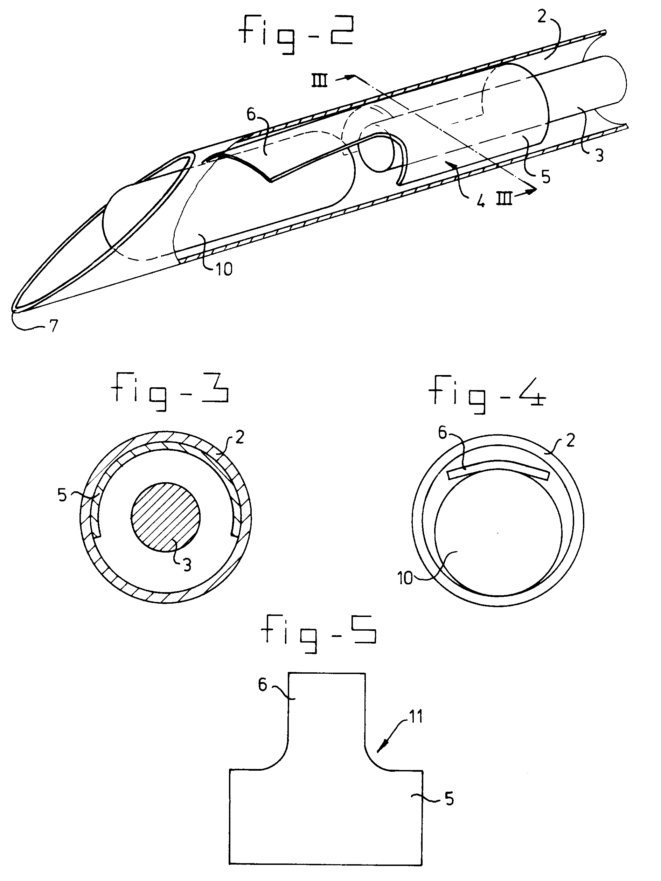

Injector 1 is provided with a casing 9 in which a mechanism is provide to convert inward movement of trigger 8 in outward movement of push rod 3 (Fig.2).

Push rod 3 is provided within hollow needle 2. Hollow needle 2 has a sharpened tip to promote 7 ingress to the skin of an animal. A cartridge 12 can be received in injector 1 and comprises a series of transponders 10 possibly surrounded by a liquid. This liquid can be a disinfecting liquid or any other medication which is desirable both with regard to protecting the hollow needle from contaminants and to promote healing of the wound of the animal after introduction of the transponder.

From cartridge 12 transponders 10 are moved through hollow needle 2 as is clear from Fig. 2. To prevent transponder 10 from dropping out of needle tip 7 during transfer of the injector to the animal, which would result in no injection at all which is not immediately clear, clamping means 4 are provided. Clamping means 4 comprise a mounting part 5 and a lip 6. In Fig. 2 the free extremity of lip 6 is urged in downward direction through the resilient nature of clamping means 4 and engages transponder 10. This is clear from Fig. 4 wherein is shown that transponder 10 is pressed against the lower side of the wall of the hollow needle. To keep the lip 6 in position mounting part 5 is provided. In its fabricated condition, as shown in Fig. 5, mounting part 5 is a simple blank of resilient material. Through bending over to an U-shape, as shown in Fig. 3, of the resilient material clamping against the side walls of the hollow needles provides a mounting effect of the sheet of resilient material is provided. The material can be of any nature, such as metal or plastic. As shown in Fig. 3 in its pinched condition the opening which is available for push rod 3 is still sufficient to prevent any restriction in its movement. This because the relatively small thickness of the material of resilient sheet 11 (Fig.5). The thickness of the sheet material can be e.g. 0,1 mm.

Although the invention is described above in an application wherein the transponder is introduced from a cartridge 12 upstream from the clamping means it is also possible to introduce a transponder from the tip 7 of the hollow needle. Because of the rounded extremities of the transponder it will easily move beyond the free extremity of lip 6 and will be clamped afterwards.

Although the invention has been described above relating to a preferred embodiment it will be obvious for the person skilled in the art that it is possible to introduce a number changes without leaving the scope of protection as defined by the appended claims. For example, it is possible to embody the mounting means as engaging only a single side wall of the hollow needle whereas it is possible to displace the clamping means by any resilient means known in the art, such as foam material and other kinds of springs.

## Claims

1. Injector for introducing solid objects (10), such as transponders, in living beings, comprising a hollow tube (2) for receiving the object and a push rod (3) displaceable in said hollow tube to transfer the object to be injected, characterized in that clamping means (4) are provided in the hollow tube to engage the object.

2. Injector according to claim 1, wherein the clamping means comprise resilient means arranged near the inner circumference of the hollow tube.

3. Injector according to claim 2, wherein the clamping means comprise a sheet of resilient material.

4. Injector according to claim 3, wherein the clamping means comprise an elongated lip (6) of sheet material having its longitudinal axis parallel to the longitudinal axis of the tube.

5. Injector according to claim 4, wherein mounting means are provided to connect the lip to the tube.

6. Injector according to claim 5, wherein the mounting means comprise a further resilient sheet part (5) connected to the lip (6) and being substantially U-shaped in the mounted position in the tube.

7. Injector according to one of the claims 5 or 6, wherein the mounting means are upstream from the lip in the tube.

8. Injector according to one or more of the claims 4-7, wherein the internal diameter remaining within the mounting means is larger than the outer diameter of the push rod.

9. Injector according to one of the preceding claims, wherein the hollow tube is provided with a sharp free extremity.

10. Sheet of resilient material, comprising a substantially rectangular base portion (5) and a projecting lip (6).
